(19) Europäisches Patentamt
European Patent Office
Office européen des brevets

(11) Publication number: **0 143 987 B1**

(12) # EUROPEAN PATENT SPECIFICATION

(45) Date of publication of patent specification: **22.02.89**

(51) Int. Cl.[4]: **A 61 K 31/70** // (A61K31/70, 31:52)

(21) Application number: **84113011.5**

(22) Date of filing: **29.10.84**

(54) **Therapeutic compositions endowed with antiviral activity.**

(30) Priority: **31.10.83 IT 2354583**

(43) Date of publication of application:
**12.06.85 Bulletin 85/24**

(45) Publication of the grant of the patent:
**22.02.89 Bulletin 89/08**

(84) Designated Contracting States:
**AT BE CH DE FR GB LI LU NL SE**

(56) References cited:
**WO-A-83/02723**
**US-A-4 199 574**

**CHEMICAL ABSTRACTS, vol. 91, no. 17, 22nd October 1979, pages 26-27, no. 133836c, Columbus, Ohio, US; E. DE CLERCQ et al.: "(E)-5-(2-bromovinyl)-2'-deoxyuridine: A potent and selective anti-herpes agent" & PROC. NATL. ACAD. SCI. U.S.A. 1979, 76(6), 2947-51**

**The file contains technical information submitted after the application was filed and not included in this specification**

(73) Proprietor: **Stendardi, Anna Gioia**
**Via Valsugana, 6**
**Cagliari (IT)**

(72) Inventor: **Stendardi, Anna Gioia**
**Via Valsugana, 6**
**Cagliari (IT)**

(74) Representative: **Gervasi, Gemma, Dr. et al**
**Studio Brevetti e Marchi NOTARBARTOLO & GERVASI 33, Viale Bianca Maria**
**I-20122 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

## Description

This invention relates to new therapeutic compositions endowed with selective antiviral activity as pointed out below more specifically.

Up to a few years ago, antiviral chemotherapy had access to only a few antiviral agents, and these possessed low or zero specificity. In recent years, numerous structural analogues of purine and pyrimidine neucleosides have been described, and some of them possess selective antiviral activity towards the herpes viruses, which are responsible for serious infections in man.

Among these new compounds, which because of their selective action have represented a considerable advance in the antiviral chemotherapy field, particularly interesting are 9-(2-hydroxyethoxymethyl) guanine (acyclovir) of formula:

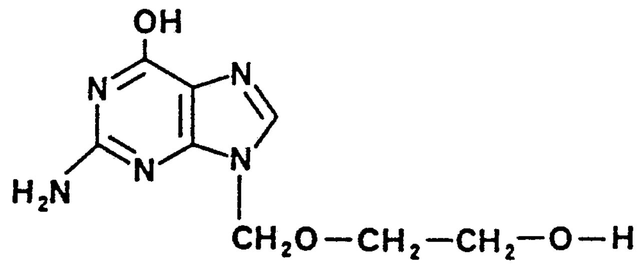

and E-5-(2-bromovinyl)-2'-deoxyuridine (BVDU), of formula:

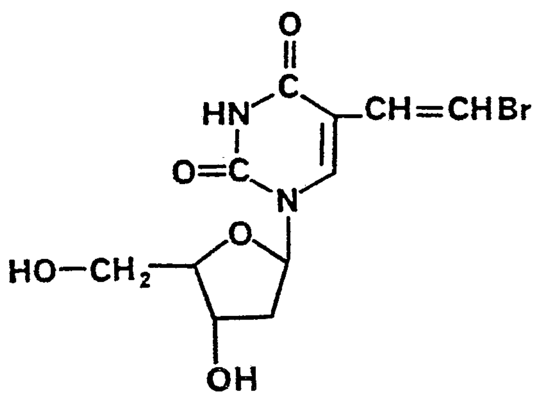

Current knowledge teaches that the selectivity of the antiviral action of acyclovir and BVDU is due to their specific interaction with two enzymes induced by the herpes viruses in the infected cells, namely pyrimidine-kinase and DNA polymerase.

Nevertheless at the practical level, the two drugs possess different spectra of antiviral activity and different degrees of toxicity. In fact, whereas acyclovir inhibits to an equal extent the multiplication of the type 1 (HSV—1) and type 2 (HSV—2) *Herpes simplex* viruses, BVDU is effective against HSV—1 but not against HSV—2. In contrast, the latter compound is considerably more active than acyclovir against the *Varicellazoster,* pseudorabies, infectious bovine Rhinotracheitis, simian Varicella and *Herpes saimiri* viruses. Next, on considering the ratio of the dose capable to inhibit the growth of uninfected cells by 50% and the dose which is effective in inhibiting the viral multiplication by 50% ($ID_{50}$), BVDU is found to have a therapeutic index (TI) which is considerably greater than that of acyclovir in all cases except HSV—2 [E. De Clerq et al. (1980) The Journal of Infectious Diseases Vol. 141, No 5; D. Bridgen et al. (1981) Antiviral Research 1 pp. 203—12; E. De Clerq (1982) Selective Antiherpes Agents, TIPS Vol. 3].

A further aspect which has to be evaluated in antiviral agents is their capacity for selecting resistant strains.

In the specific case of acyclovir and BVDU, it is known that resistant mutants are promptly selected in vitro even following treatment with doses which are 1000 times greater than the respective $ID_{50}$ doses. This also occurs in vivo, given that resistant herpes viruses are always more frequently isolated from patients being treated with one or the other drug [W. H. Burns et al. (1982) Lancet 421—423; D. J. Coster et al. (1979) Br. J. Ophthalmol. Vol. 63 pp. 418—421; C. S. Crumpaker et al. (1982) N. Engl. J. Med. Vol. 306 pp. 343—346].

In conclusion, acyclovir and BVDU are two drugs which can be used with good results in herpetic infections. However, their therapeutic indices are not satisfactory in all cases, and both very easily induce the selection of resistant mutants.

The aim of the present invention is to provide new therapeutic compositions made up from acyclovir and BVDU combined in certain proportions so as to result either in an antiviral drug possessing the spectrum of acyclovir but an antiviral activity strongly potentiated by synergism because of the presence of BVDU in given proportions, or in a drug possessing the spectrum of BVDU, but having an antiviral activity strongy potentiated by synergism because of the presence of acyclovir in given proportions.

The new antiviral compositions according to the invention also have a clinical reliability greater than that of the individual constituents, since they reduce the emergence of resistant mutants.

As a matter of fact, the possibility of attaining said objects by means of the combination of selective antivirals, and in particular by the acyclovir-BVDU association, has already been examined [R. F. Schinazi et al. (1982) and K. K. Biron et al. (1982), in The American Journal of Medicine — Acyclovir Symposium].

From tests carried out by known methods, based on associating all the known antivirals with each other, the conclusion was drawn that the combination of two antiviral drugs can rise to either additive, indifferent, antagonistic or synergistic effects in a manner which is absolutely unpredictable because of the still incomplete knowledge of the relative modes of action. In the case of the acyclovir-BVDU association, the authors of the known art reached the conclusion that, when acyclovir and BCDU are mixed in any proportion, they always produce an additive effect (Schinazi et al. page 45, column 2; Biron et al. page 56, column 2).

Moreover, even though one of the premises offered in support of the therapeutic validity of associating antiviral drugs was the possibility of reducing the emergence of resistant viral strains, in no case was such an occurrence experimentally documentated.

What we have now discovered, namely that combinations of acyclovir and BVDU, within a well defined range of proportions, have an antiviral action strongly potentiated with respect to the starting compounds by virtue of synergistic effect, thus constitutes not only a considerable technical progress in the antiviral chemotherapy field, but also an unpredictable overcoming of technical prejudice.

The present invention provides pharmaceutical compositions endowed with selective antiviral activity, constituted by acyclovir and BVDU combined in molar ratios of between 50:1 and 1:5.

None of the compositions according to the invention has been proved toxic for uninfected cell cultures.

Moreover, the new compositions have demonstrated a capacity for selecting resistant mutants which is considerably lower than that determined for the individual starting compounds.

The aim and the results attained by the present invention will be more evident from the analysis of some significant experimental results.

The mutant synergistic activity of acyclovir and BVDU was evaluated on the *Herpes simplex* virus type 1 (ATCC VR—733), grown in Vero cells (ATCC CCL 81) in MEM T—199 containing 2% calf serum.

The method used was the plaque reduction test [P. Collins et al. (1977) Annals of the New York Academy of Sciences 284, pp. 49—59]. For this purpose, confluent monolayers of Vero cells, seeded in multi-well plates (diameter 3.5 cm) at a concentration of $3 \times 10^6$ cells/plate, were infected with 150 plaque-forming units (PFU) of HSV—1 per plate.

After an adsorption period of one hour at room temperature and removal of the unadsorbed virus, the monolayers were washed with buffered saline solution and then incubated at 35°C, in 5% $CO_2$, in MEM T—199 containing 2% calf serum and 0.75% carboxymethylcellulose, both in the absence and in the presence of the inhibitors.

72 hours later, the infected monolayers were washed three times with saline solution and dyed with a 0.1% methylene blue solution in order to be able to count the number of lysis plaques resulting from the multiplication of the virus.

The results are shown in Table 1. The number represent the mean value of three samples, and correspond to the number of plaques obtained in the absence or in the presence of acyclovir and BVDU, used either individually or in association.

TABLE 1

| | BVDU (μM) | | | | |
|---|---|---|---|---|---|
| Acyclovir (μM) | 0 | 0.8 | 0.04 | 0.02 | 0.01 |
| 0 | 134 | 81 | 109 | 131 | 141 |
| 0.075 | 111 | 53 | 86 | 88 | 111 |
| 0.15 | 99 | 29 | 69 | 61 | 82 |
| 0.3 | 52 | 10 | 26 | 58 | 54 |
| 0.6 | 8 | 0 | 4 | 2 | 6 |
| 1.2 | 0 | 0 | 1 | 3 | 1 |

The above values were then expressed as percentage of the number of plaques obtained in untreated control cultures and plotted, in the ordinate axis, against the logarithm of the concentration used (in abscissa).

This enabled to determine the concentration of the two drugs (alone or in combination) which were necessary for reducing the number of viral plaques by 100% ($ID_{100}$).

Two methods, namely a graphical method and a mathematical method, were used to evaluate the type of effect obtained with the different combinations of acyclovir and BVDU.

The first method consists of plotting on an arithmetic scale the quantities of acyclovir and BVDU, both alone and in the various combinations, which produce a same effect, i.e. 100% inhibition of plaque development. If the combinations produce a simply additive effect, the relative $ID_{100}$ values fall on the straight line joining the $ID_{100}$ values for acyclovir and BVDU used individually. If the combinations produce a synergistic effect, the values fall below the line.

The mathematical method consists of calculating the FIC (fractional inhibitory concentration) indices from the following formula:

$$\text{FIC index} = \frac{ID_{100}\text{ acyclovir in combination}}{ID_{100}\text{ acyclovir alone}} + \frac{ID_{100}\text{ BVDU in combination}}{ID_{100}\text{ BVDU alone}}$$

If the FIC index is equal to 1, the effect produced by the combination is additive. If is is lower than 1, the effect is synergistic to an extent which is greater the smaller is the value of the FIC index. The results obtained with both these methods are shown in the isobologram of Figure 1.

As can be seen, the acyclovir and BVDU concentration necessary to reduce the number of HSV—1 plaques by 100%, when the two drugs are used individually, are 0.75 μM, and 0.7μM, respectively. When acyclovir and BVDU are used in association, in ratios of between 50:1 and 1:5, 100% inhibition of viral development is obtained at concentrations of both drugs, which are much lower than those expected for a simply additive effect. Furthermore, the synergistic effect of the combinations is demonstrated by the fact that all the FIC index values are much lower than 1. Maximum synergism is obtained for acyclovir and BVDU concentrations of between 2:1 and 5:1.

Three significant samples from the therapeutic compositions shown in Table 1 were also tested in order to evaluate their capacity to interfere with the exponential multiplication of uninfected cells. The results are shown in Figure 2.

This shows that none of the combinations tested is able to impair the exponential growth of Vero cells exposed to their action for five days.

Finally, tests were carried out which aimed at quantifying the appearance of resistant HSV—1 mutants following treatment with acyclovir and BVDU, used individually or in association. For those tests the two compounds were used in concentrations which were 3.3. times (acyclovir) and 1.4 times (BVDU) the $ID_{100}$ value, so as to be able to completely prevent the multiplication of sensitive viruses. For this purpose, $5 \times 10^5$ Vero cells, infected with HSV—1 at a multiplicity of 0.3 PFU/cell, were seeded on a preformed monolayer of uninfected Vero cells and left to adhere for 4 hours at 35°C in T—199 with 2% serum, in the presence of the inhibitors.

The liquid culture medium was then removed and replaced with new medium containing the inhibitors and 0.75% carboxymethylcellulose. The number of plaques developed following multiplication of resistant viruses was determined 72 hours later.

From the results shown in Table 2 it is apparent that HSV—1 mutants resistant to acyclovir and BVDU pre-exist within the virus population in amounts close to 3 and 1 per 1000 sensitive viruses, respectively.

However, when one of the two drugs (in a concentration much below the $ID_{100}$) is associated with the other (used at a concentration able to select resistant mutants), the development of said mutants is drastically reduced.

TABLE 2

| Compounds | Resistant mutants/$10^4$ viruses |
|---|---|
| Acyclovir 2.5 μM | 28.6 |
| Acyclovir 2.5 μM + BVDU 0.2 μM | 3.0 |
| Acyclovir 2.5 μM + BVDU 0.1 μM | 7.8 |
| Acyclovir 2.5 μM + BVDU 0.05 μM | 16.2 |
| BVDU 1.0 μM | 13.8 |
| BVDU 1.0 μM + acyclovir 0.4 μM | 3.27 |
| BVDU 1.0 μM + acyclovir 0.2 μM | 6.7 |
| BVDU 1.0 μM + acyclovir 0.1 μM | 8.3 |

Acyclovir is currently used clinically in the treatment of kerato-conjunctivitis and stomatitis from HSV—1, genital infections from HSV—2, and zoster.

BVDU is in advanced state of clinical experimentation for kerato-conjunctivitis and stomatitis form HSV—1, and for zoster.

The present invention thus provides means for curing said affections which are considerably more effective, less toxic and more reliable in that they reduce the emergence of resistant mutants.

## Claims

1. Therapeutic compositions possessing an antiviral activity potentiated by synergism with respect to the individual components, constituted by 9-(2-hydroxyethylmethyl) guanine (acyclovir) of formula:

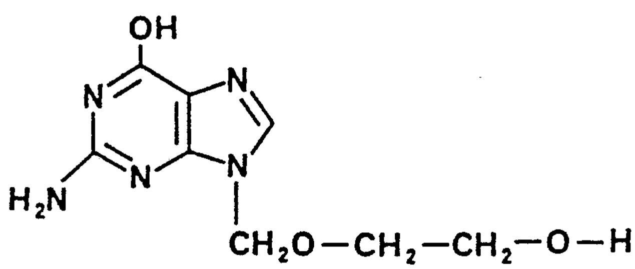

and E-5-(2-bromovinyl)-2'-deoxyuridine (BVDU), in a molar ratio of between 50:1 and 1:5.

2. Therapeutic compositions as claimed in claim 1, constituted by acyclovir potentiated by synergism by the addition of BVDU in an acyclovir/BVDU molar ratio of between 5:1 and 2:1.

## Patentansprüche

1. Therapeutische Zusammensetzungen, die in bezug auf die einzelnen Bestandteile durch Synergismus verstärkte antivirale Wirksamkeit aufweisen, bestehend aus 9-(2-Hydroxyäthoxymethyl)-guanin (Acyclovir) der Formel

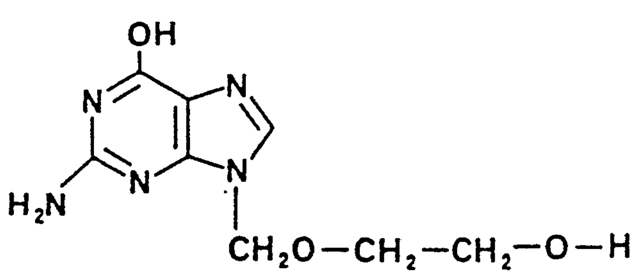

und E-5-(2-Bromvinyl)-2'-desoxyuridan (BVDU) im Molverhältnis zwischen 50:1 und 1:5.

2. Therapeutische Zusammensetzungen, wie in Anspruch 1 beansprucht, bestehend aus Acyclovir verstärkt durch Synergismus durch den Zusatz von BVDU in einem Molverhältnis von Acyclovir/BVDU zwischen 5:1 und 2:1.

**Revendications**

1. Compositions thérapeutiques possédant une activité antivirale renforcé par synergie par rapport aux composants individuels, constituées de 9-(2-hydroxyéthoxyméthyl)guanine (acyclovir) de formule:

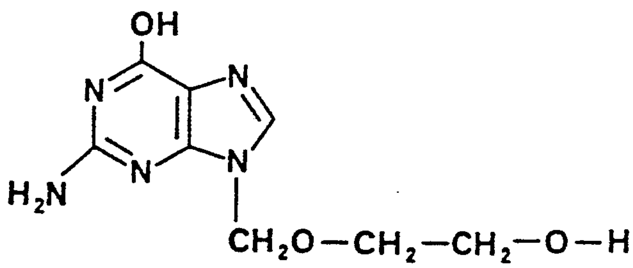

et de E-5-(2-bromovinyl)-2'-désoxyuridine (BVDU), selon un rapport en moles compris entre 50:1 et 1:5.

2. Compositions thérapeutiques selon la revendiction 1, constituées d'acyclovir renforcé par synergie par addition de BVDU selon un rapport en moles acyclovir/BVDU compris entre 5:1 et 2:1.

FIG. 1

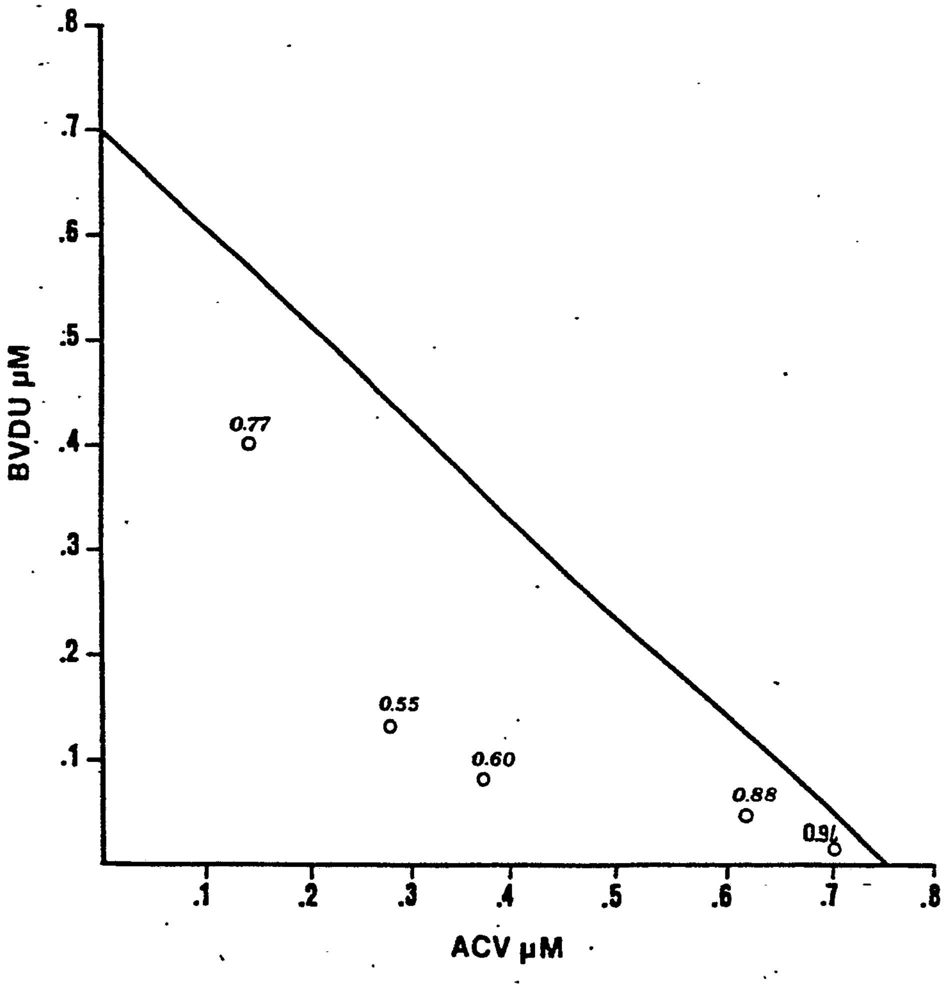

FIG. 2

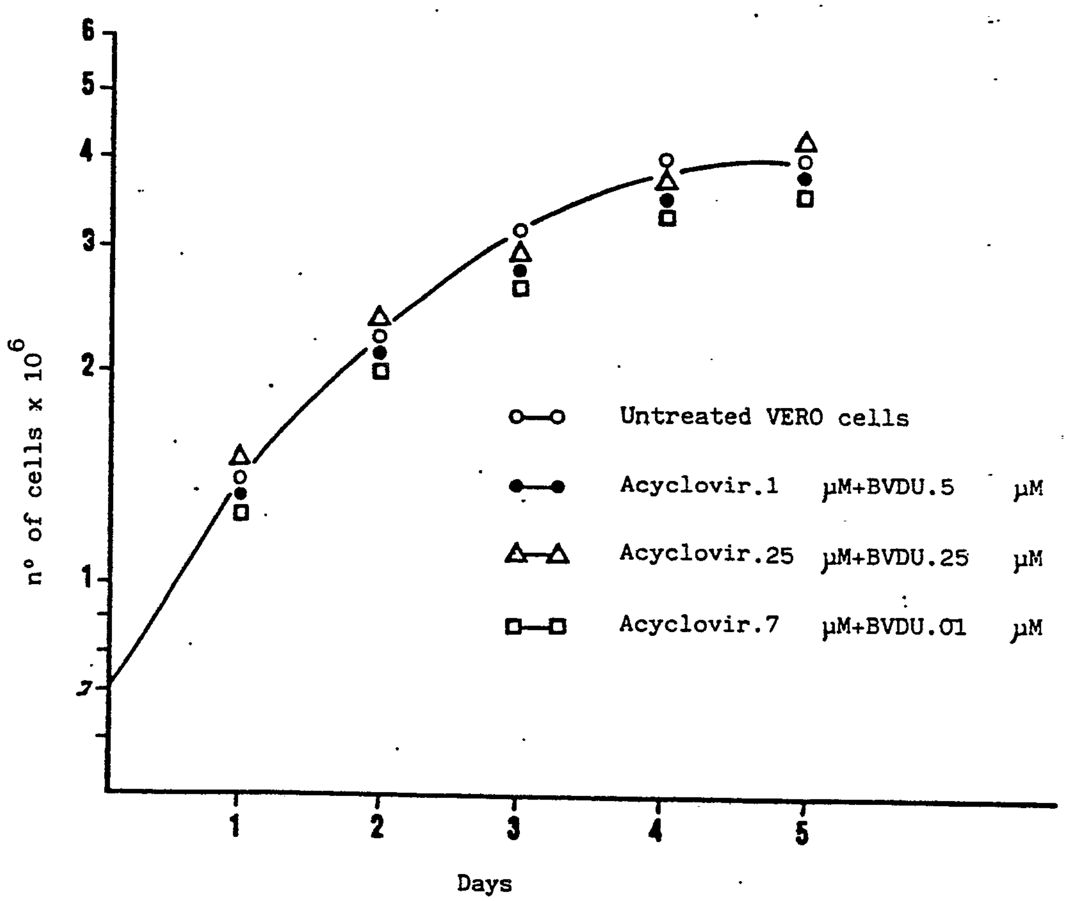
6
5
4
3
2
1
.7
n° of cells x 10^6
1
2
3
4
5
Days
Untreated VERO cells
Acyclovir.1 μM+BVDU.5 μM
Acyclovir.25 μM+BVDU.25 μM
Acyclovir.7 μM+BVDU.01 μM